(19) **Europäisches Patentamt**
**European Patent Office**
**Office européen des brevets**

(11) **EP 2 226 061 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**25.01.2017 Patentblatt 2017/04**

(45) Hinweis auf die Patenterteilung:
**26.12.2012 Patentblatt 2012/52**

(21) Anmeldenummer: **09003321.8**

(22) Anmeldetag: **06.03.2009**

(51) Int Cl.:
***A61K 6/083*** (2006.01)

(54) **Infiltrant für die Dentalapplikation**

Infiltrant for dental application

Infiltrant pour l'application dentaire

(84) Benannte Vertragsstaaten:
**CH DE FR GB LI**

(43) Veröffentlichungstag der Anmeldung:
**08.09.2010 Patentblatt 2010/36**

(73) Patentinhaber: **Ernst Mühlbauer GmbH & Co.KG 25870 Norderfriedrichskoog (DE)**

(72) Erfinder:
- **Neffgen, Stephan 22459 Hamburg (DE)**
- **Neander, Swen 20148 Hamburg (DE)**

(74) Vertreter: **Glawe, Delfs, Moll Partnerschaft mbB von Patent- und Rechtsanwälten Postfach 13 03 91 20103 Hamburg (DE)**

(56) Entgegenhaltungen:
EP-A- 1 913 927          WO-A-2007/131725
JP-A- H01 230 508     JP-A- 2005 213 207

- **MEYER-LUECKEL H ET AL: "Influence of the application time on the penetration of different dental adhesives and a fissure sealant into artificial subsurface lesions in bovine enamel" DENTAL MATERIALS, ELSEVIER, Bd. 22, Nr. 1, 1. Januar 2006 (2006-01-01), Seiten 22-28, XP025089581 ISSN: 0109-5641 [gefunden am 2006-01-01]**
- **PARIS S. ET AL: 'Penetration coefficients of commercially available and experimental composites intended to infiltrate enamel carious lesions' DENTAL MATERIALS Bd. 23, 2007, Seiten 742 - 748**
- **VAN LANDUYT K.L. ET AL: 'Systematic review of the chemical composition of contemporary dental adhesives' BIOMATERIALS Bd. 28, 2007, Seiten 3757 - 3785**
- **Technical Manual "Prime & Bond NT" from Dentsply De Trey GmbH**
- **BELOICA M. ET AL: 'Efficacy of all-in-one adhesive systems on unground enamel' INTERNATIONAL DENTISTRY SA Bd. 10, Nr. 5, Seiten 12-14 - 16;48**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft einen Infiltranten für die Dentalapplikation, der vernetzende Monomere enthält zur Anwendung in der Behandlung bzw. Prävention von kariösen Schmelzläsionen.

Unter kariösen Schmelzläsionen sind hier im Wesentlichen sich im Zahnschmelz ausdehnende kariöse Schädigungen zu verstehen, die noch nicht zur Kavitation (Lochbildung) geführt haben. Kariöse Schmelzläsionen sind demineralisierte Bereiche des Zahnschmelzes, die eine Tiefe bis zu etwa 2-3 mm haben können.

Aus der veröffentlichten Internationalen Anmeldung WO 2007/131725 A1 sind zur Behandlung kariöser Schmelzläsionen ein Infiltrationsverfahren und Infiltranten bekannt, so dass die Kavitation verhindert wird und sich die sonst üblicherweise erfolgende Restauration mit Dentalkompositen erübrigt. Bei dem Infiltrationsverfahren wird, nachdem eine evtl. vorhandene remineralisierte oberflächliche Schicht entfernt wurde, die Läsion mit einem Infiltranten, der im Wesentlichen aus Monomeren besteht, in Kontakt gebracht, woraufhin diese infiltrieren. Nachdem der Infiltrant die Läsion durchdrungen hat, werden die Monomere mittels Lichtaktivierung polymerisiert. Die Läsion ist dann versiegelt. Das Fortschreiten der Karies wird gestoppt.

Für die Infiltration sind spezielle Monomere bzw. Monomermischungen erforderlich, da bekannte dentale Haftvermittler, Dentalkleber oder Dentallacke (auch Primer, Adhäsive, Bondings oder Sealants genannt) zu langsam und/oder nicht ausreichend in die Läsion eindringen und/oder die Läsion vollständig durchdringen (penetrieren oder infiltrieren) und/oder nicht vollständig und/oder schnell durchhärten (polymerisieren). Die WO 2007/131725 A1 beschreibt die Verwendung Monomerer bzw. Monomermischungen, so dass der Infiltrant einen Penetrationskoeffizienten PK > 50 hat.

Nachteilig an den dort beschrieben Infiltranten sind deren Erhärtungseigenschaften; insbesondere die Durchhärtungstiefe, sowie die mechanischen Eigenschaften des polymerisierten Infiltranten, insbesondere die Verbundfestigkeit mit der Zahnsubstanz wie bspw. dem Zahnschmelz sowie die Spannungsrissstabilität.

Der Erfindung liegt die Aufgabe zugrunde, einen Infiltranten der eingangs genannten Art zu schaffen, der verbesserte Erhärtungseigenschaften und eine gute Versiegelungswirkung aufweist sowie langlebig ist.

Die Erfindung ist in den Patentansprüchen definiert. Der erfindungsgemäße Infiltrant weist einen Penetrationskoeffizienten PK > 100 cm/s auf und enthält 0,05 - 20 Gew.-% säuregruppenhaltige Monomere.

Zunächst seien einige im Rahmen der Erfindung verwendete Begriffe erläutert. Der Begriff Infiltrant bezeichnet eine Flüssigkeit, die als ungehärtetes Harz in eine Zahnschmelzläsion (einen porösen Festkörper) eindringen kann. Nach dem Eindringen kann der Infiltrant dort ausgehärtet werden.

Vernetzende Monomere weisen zwei oder mehr polymerisierbare Gruppen auf und können daher bei einer Polymerisation polymerisierte Ketten miteinander vernetzen.

Das Eindringen einer Flüssigkeit (ungehärtetes Harz) in einen porösen Festkörper (Zahnschmelzläsion) wird durch die Washburn-Gleichung (Gleichung 1, siehe unten) physikalisch beschrieben. Bei dieser Gleichung wird angenommen, dass der poröse Festkörper ein Bündel offener Kapillaren darstellt (Buckton G., Interfacial phenomena in drug delivery and targeting. Chur, 1995); in diesem Fall wird das Eindringen der Flüssigkeit von Kapillarkräften getrieben.

$$d^2 = \left( \frac{\gamma \cdot \cos\theta}{2\eta} \right) r \cdot t \qquad \text{– Gleichung 1 –}$$

d    Abstand, um den sich das flüssige Harz bewegt
$\gamma$    Oberflächenspannung des flüssigen Harzes (gegen Luft)
$\theta$    Kontaktwinkel des flüssigen Harzes (gegen Zahnschmelz)
$\eta$    dynamische Viskosität des flüssigen Harzes
$r$    Kapillarradius (Porenradius)
$t$    Eindringdauer

Der in Klammern stehende Ausdruck der Washburn-Gleichung wird als Penetrationskoeffizient (PK, Gleichung 2, siehe unten) bezeichnet (Fan P. L. et al., Penetrativity of sealants. J. Dent. Res., 1975, 54: 262-264). Der PK setzt sich aus der Oberflächenspannung der Flüssigkeit gegen Luft ($\gamma$), dem Cosinus des Kontaktwinkels der Flüssigkeit gegen Zahnschmelz ($\theta$) und der dynamischen Viskosität der Flüssigkeit ($\eta$) zusammen. Je größer der Wert des Koeffizienten ist, umso schneller dringt die Flüssigkeit in eine gegebene Kapillare oder ein gegebenes poröses Bett ein. Dies bedeutet, dass ein hoher Wert des PK durch hohe Oberflächenspannungen, niedrige Viskositäten und niedrige Kontaktwinkel erzielt werden kann, wobei der Einfluss des Kontaktwinkels vergleichsweise gering ist.

$$PK = \left( \frac{\gamma . \cos \theta}{2\eta} \right) \quad - \text{Gleichung 2} -$$

*PK*     Penetrationskoeffizient

$\gamma$     Oberflächenspannung des flüssigen Harzes (gegen Luft)

$\theta$     Kontaktwinkel des flüssigen Harzes (gegen Zahnschmelz)

$\eta$     dynamische Viskosität des flüssigen Harzes

Infiltranten werden häufig in schwer zugänglichen Zahnzwischenräumen (approximal) angewendet. Insbesondere bei der Verwendung lichthärtender Systeme ist eine optimale Bestrahlung zur Durchführung der Aushärtung in Zahnzwischenräumen schwierig. Die Erfindung hat erkannt, dass sich durch die Verwendung einer in Anspruch 1 definierten Zusammensetzung eine hinreichende Durchhärttiefe auch unter den genannten schwierigen Bedingungen erreichen lässt und andererseits ein hinreichend hoher Penetrationskoeffizient erhalten bleiben kann, der eine ausreichende Eindringtiefe des Harzes sicherstellt. Die erfindungsgemäße Zusammensetzung des Infiltranten bewirkt somit eine gute Eindringfähigkeit kombiniert mit einer guten Aushärtbarkeit auch unter den schwierigen Bedingungen einer approximalen Applikation.

Bevorzugt liegt der Penetrationskoeffizient des Infiltranten über 150 cm/s, er kann aber auch über 200 cm/s liegen.

Der erfindungsgemäß vorgesehene Zusatz säuregruppenhaltiger Monomere erhöht nicht nur die Durchhärtetiefe, sondern verbessert auch die Haftung am Zahnmaterial wie bspw. Zahnschmelz. Weiterhin kann der Gesamtgehalt an vernetzenden insbesondere mehr als 2 polymerisierbare Gruppen enthaltenden Monomeren gesenkt werden, was zu einer verbesserten Spannungsrissstabilität führt. Durch den erfindungsgemäßen Zusatz der säuregruppenhaltigen Monomere wird der dauerhafte Verschluss der Läsion weiter verbessert. Die Sperrwirkung gegen Diffusion niedermolekuarer Nährstoffe, bspw. Kohlenhydrate (Zucker), wird weiter erhöht. Der Gehalt an säuregruppenhaltigen Monomeren beträgt vorzugsweise 0,1 bis 15 Gew.-%, weiter vorzugsweise 0,2 bis 10 Gew.-%, weiter vorzugsweise 0,5 bis 5 Gew.-%, weiter vorzugsweise 0,5 bis 2 Gew.-%.

Geeignete Säuregruppen sind bspw. Carbon- und Sulfonsäuregruppen. Bevorzugte Säuregruppen in den säuregruppenhaltigen Monomeren sind Phosphor und/oder Phosphonsäuregruppen. Beispielhaft genannt seien entsprechende organische Hydrogenphosphate oder Dihydrogenphosphate.

Die säuregruppenhaltigen Monomere können vernetzend ausgebildet sein, also außer der Säuregruppe noch zwei oder mehr polymerisierbare Gruppen enthalten, bspw. dann Monomere der Formel

$$R^1 \, [X_k \, R^2_1 \, Y_m]_n .$$

In ausgewählten Anwendungen kann es vorteilhaft sein, wenn sie lediglich eine polymerisierbare Gruppe enthalten. Insbesondere können die säuregruppenhaltigen Monomere Acrylate oder Methacrylate, Acrylamide oder Methacrylamide sein.

Beispielsweise kann es sich um Acrylate oder Methacrylate handeln, die weiter unten aufgezählt sind als bevorzugte zusätzlich enthaltene Monomere mit einer polymerisierbaren Gruppe und die zusätzlich eine entsprechende Säuregruppe aufweisen.

Die säuregruppenhaltigen Monomere haben erfindungsgemäß bevorzugt ein Molekulargewicht (Gewichtsmittel) von 100 - 1000 g/mol. Bevorzugt sind die säuregruppenhaltigen Monomere in den nicht wässrigen Harzmischungen des Infiltranten löslich. Der Begriff "nicht wässrig" bezeichnet dabei vorzugsweise Harzmischungen, die weniger als 5 Gew.-%, bevorzugt weniger als 2 Gew.-% Wasser enthalten.

Bevorzugt verwendbare säuregruppenhaltigen Monomere sind beispielsweise 4-Methacryloxyethyltrimellitsäure (4-MET), Methacryloyloxydecylmalonsäure (MAC-10), Maleinsäuremono-HEMA-ester, N-Methacryloyl-N',N'-dicarboxymethyl-1,4-diaminobenzen, N-2-Hydroxy-3-methacryloyloxypropyl-N-phenylglycin, O-Methacryloyltyrosinamid, 4-Methacryloylaminosalicylsäure, Phenylmethacryloxyalkylphosphate, bspw. Phenylmethacryloxyethylphosphat (Phenyl-P), Methacryloyloxyalkyldihydrogenphosphate, bspw. Methacryloyloxyethyldihydrogenphosphat (HEMA-P), Methacryloyloxypropyldihydrogenphosphat (MPP), Methacryloyloxyhexyldihydrogenphosphat (MHP), Methacryloyloxydecyldihydrogenphosphat (MDP), Glyceryldimethacrylatphosphat (GPDM), Pentaerythritoltriacrylatphosphat (PENTA-P), Bis(hydroxyethylmethacrylat)phosphat, (Meth)acrylamidophosphate, (Meth)acrylamidodiphosphate, (Meth)acrylamidoalkylphosphonate, (Meth)acrylamidoalkyldiphosphonate, Bismethacrylamidoalkyldihydrogenphosphate, Vinylbenzylphosphonsäure und Vinylbenzoesäure.

Unter den genannten Methacryloyloxyalkyldihydrogenphosphaten ist Methacryloyloxydecyldihydrogenphosphat (MDP) besonders bevorzugt.

Die erfindungsgemäßen Infiltranten lassen sich je nach chemischem Aufbau der enthaltenen Monomere radikalisch, anionisch oder kationisch härten. Bevorzugt sind die Monomere radikalisch oder kationisch härtbar.

Die radikalische Härtung der erfindungsgemäßen Monomere kann durch Vinylpolymerisation geeigneter Doppelbindungen erfolgen. Besonders geeignet sind hier (Meth)acrylate, (Meth)acrylamide wie sie bspw. in der EP 1674066 A1 und der EP 1974712 A1 offenbart sind, Styrylverbindungen, Cyanacrylate und Verbindungen mit ähnlich gut radikalisch polymerisierbaren Doppelbindungen. Eine weitere Möglichkeit der radikalischen Härtung besteht in der ringöffnenden Polymerisation von cyclischen Vinylverbindungen wie den in EP1413569, EP1741419 und EP1688125 beschriebenen Vinylcyclopropanen oder anderen cyclischen Systemen wie Vinylidensubstituierten Orthospirocarbonaten oder Orthospiroestern. Eine weitere Möglichkeit besteht auch in der Copolymerisation der ringöffnend polymerisierenden Systeme mit den oben genannten einfach polymerisierenden Doppelbindungen.

Die radikalische Härtung kann darüber hinaus durch eine unter dem Begriff Thiol-En-Reaktion bekannte Stufenreaktionen, wie in WO 2005/086911 beschrieben, erfolgen.

Die kationische Härtung der erfindungsgemäßen Monomere kann ebenfalls durch ringöffnende wie auch durch Vinylpolymerisation erfolgen. Geeignete Vinylpolymere sind Vinylether, Styrylverbindungen und weitere Verbindungen mit elektronenreichen Vinylgruppen. Geeignete ringöffnend polymerisierende Monomere sind Verbindungen, die Epoxid-, Oxetan-, Aziridin-, Oxazolin oder Dioxolangruppen tragen. Weitere ringöffnend polymerisierende Gruppen können der Literatur; bspw.: K.J. Ivin, T. Saegusa, (Eds.), Vol2, Elsevier Appl. Sci. Publ., London 1984; entnommen werden. Besonders geeignet sind siliziumhaltige Epoxidmonomere, wie sie in WO 02/066535 oder WO 2005/121200 beschrieben sind. Besonders vorteilhaft bei der Verwendung von Epoxiden oder Oxetanen ist der geringe Polymerisationsschrumpf wie auch die geringe Inhibitionsschicht dieser Materialien.

Der Anteil vernetzender Monomere mit zwei polymerisierbaren Gruppen beträgt 99,8 bis 50 Gew.-%, vorzugsweise 99,5 bis 60 Gew.-%, weiter vorzugsweise 99 bis 60 Gew.-%, weiter vorzugsweise 99 bis 70 Gew.-%.

Die vernetzenden Monomere mit zwei polymerisierbaren Gruppen sind bevorzugt Derivate der Acryl- und/oder Methacrylsäure. Sie können vorzugsweise ausgewählt sein aus der Gruppe bestehend aus Allylmethacrylat; Allylacrylat; PRDMA, 1,3-Propandioldimethacrylat; BDMA, 1,3-Butandioldimethacrylat; BDDMA, 1,4-Butandioldimethacrylat; PDDMA, 1,5-Pentandioldimethacrylat; NPGDMA, Neopentylglykoldimethacrylat; HDDMA, 1,6-Hexandioldimethacrylat; NDDMA, 1,9-Nonandioldimethacrylat; DDDMA, 1,10-Decandioldimethacrylat; DDDDMA, 1,12-Dodecandioldimethacrylat; PRDA, 1,3-Propandioldiacrylat; BDA, 1,3-Butandioldiacrylat; BDDA, 1,4-Butandioldiacrylat; PDDA, 1,5-Pentandioldiacrylat; NPGDA, Neopentylglykoldiacrylat; HDDA, 1,6-Hexandioldiacrylat; NDDA, 1,9-Nonandioldiacrylat; DDDA, 1,10-Decandioldiacrylat; DDDDA, 1,12-Dodecandioldimethacrylat; EGDMA, Ethylenglykoldimethacrylat; DEGDMA, Diethylenglykoldimethacrylat; TEDMA, Triethylenglykoldimethacrylat; TEGDMA, Tetraethylenglykoldimethacrylat; EGDA, Ethylenglykoldiacrylat; DEGDA, Diethylenglykoldiacrylat; TEDA, Triethylenglykoldiacrylat; TEGDA, Tetraethylenglykoldiacrylat; PEG200DMA, Polyethylenglykol 200 Dimethacrylat; PEG300DMA, Polyethylenglykol 300 Dimethacrylat; PEG400DMA, Polyethylenglykol 400 Dimethacrylat; PEG600DMA, Polyethylenglykol 600 Dimethacrylat; PEG200DA, Polyethylenglykol 200 Diacrylat; PEG300DA, Polyethylenglykol 300 Diacrylat; PEG400DA, Polyethylenglykol 400 Diacrylat; PEG600DA, Polyethylenglykol 600 Diacrylat; PPGDMA, Polypropylenglykoldimethacrylat; PPGDA, Polypropylenglykoldiacrylat; NPG(PO)2DMA, Propoxyliertes (2) Neopentylglykoldimethacrylat; NPG(PO)2DA, Propoxyliertes (2) Neopentylglykoldiacrylat; Bis-MA, Bisphenol-A-dimethacrylat; Bis-GMA, Bisphenol-A-glyceroldimethacrylat; BPA(EO)DMA, Ethoxyliertes Bisphenol-A-Dimethacrylat(EO=1-30); BPA(PO)DMA, Propoxyliertes Bisphenol-A-Dimethacrylat(PO=1-30); BPA(EO)DA, Ethoxyliertes Bisphenol-A-Diacrylat(EO=1-30); BPA(PO)DA, Propoxyliertes Bisphenol-A-Diacrylat(PO=1-30); BPA(PO)GDA, Propoxyliertes Bisphenol-A-glyceroldiacrylat; UDMA, Diurethandimethacrylat; TCDDMA, Tricyclo[5.2.1.0]decandimethanoldimethacrylate; TCDDA, Tricyclo[5.2.1.0]decandimethanoldiacrylate; EBA, N,N'-Ethylenbisacrylamid; DHEBA, N,N'-(1,2-Dihydroxyethylen)bisacrylamid; DEPBA, N,N'-Diethyl(1,3-propylen)bisacrylamid; TMHMBMA, N,N'-(2,2,4-Trimethylhexamethylen)bismethacrylamid; und Bis[2-(2-methylacrylamino)ethoxycarbonyl]hexamethylendiamin.

Der Anteil vernetzender Monomere mit mindestens drei polymerisierbaren Gruppen kann vorzugsweise zwischen 0 und 50 Gew.-%, weiter vorzugsweise 10 und 30 Gew.-% liegen.

Bevorzugte vernetzende Monomere mit mindestens drei polymerisierbaren Gruppen weisen die nachfolgende Formel

$$R^1[X_kR^2_1Y_m]_n$$

auf, mit folgenden Bedeutungen

$R^1$ ist ein linearer oder verzweigter Kohlenwasserstoff mit 3-24 C-Atomen, enthaltend Alkyl, Cycloalkyl oder Aryl;

optional enthaltend O, N, Si, S, P als Heteroatome, beispielsweise Siloxan und/oder Cyclosiloxan und/oder Carbosilan und/oder Cyclocarbosilan und/oder insbesondere Ether- oder Polyethergruppen, Polyestergruppen, Polysilo-

4

xangruppen oder Polycarbosilangruppen;

optional substituiert durch Hydroxyl und/oder Carbonyl und/oder Halogen (bevorzugt Fluor) und/oder Ammoniumalkylengruppen und/oder Siloxan und/oder Cyclosiloxan und/oder Carbosilan und/oder Cyclocarbosilan;

$R^2$ ist ein linearer oder verzweigter Kohlenwasserstoff mit 1-16 C-Atomen, enthaltend Alkyl, Cycloalkyl oder Aryl;

optional enthaltend O, N, Si, S, P als Heteroatome, beispielsweise Siloxan und/oder Cyclosiloxan und/oder Carbosilan und/oder Cyclocarbosilan und/oder insbesondere Ether- oder Polyethergruppen, Polyestergruppen, Polysiloxangruppen oder Polycarbosilangruppen;

optional substituiert durch Hydroxyl und/oder Carbonyl und/oder Halogen (bevorzugt Fluor) und/oder Ammoniumalkylengruppen und/oder Siloxan und/oder Cyclosiloxan und/oder Carbosilan und/oder Cyclocarbosilan;

X ist eine verknüpfende Gruppe gleich oder verschieden ausgewählt aus einer Ethergruppe, Carbonylgruppe, Estergruppe, Amidgruppe, Urethangruppe oder Harnstoffgruppe;

Y ist eine Gruppe gleich oder verschieden enthaltend eine polymerisierbare Doppelbindung und/oder eine ringöffnend polymerisierbare Gruppe und/oder eine Thiolgruppe und/oder eine Epoxidgruppe und/oder eine Vinyl, bevorzugt (Meth)acrylat und/oder (Meth)acrylamid;

k  ist 0 oder 1;

l  ist 0 oder 1

m  ist mindestens 1;

n  ist mindestens 1

$m \times n$ ist mindestens 3.

Es kann von Vorteil sein, wenn die Monomeren zusätzliche funktionelle Gruppen aufweisen wie Ammoniumalkylengruppen oder Halogen, insbesondere fluoriertes Alkylen.

Geeignete niedrigviskose Monomeren mit mindestens drei polymerisierbaren Gruppen sind beispielsweise Glyceroltriacrylat, TMPTMA, Trimethylolpropantrimethacrylat, TMPTA, Trimethylolpropantri(meth)acrylat; DTMPTA; Di-Trimethylolpropantetra(meth)acrylat; DiPENTA, Di-Pentaerythritolpenta(meth)acrylat; oder DPEHA, Di-Pentaerythritolhexa(meth)acrylat.

Bevorzugte niedrigviskose Monomere mit mindestens drei polymerisierbaren Gruppen basieren bspw. auf alkoxylierten Mehrfachalkoholen (Tri, Tetra-, Penta, Hexa-, Polyole)wie Trimethylolpropan, Ditrimethylolpropan, Glycerol, Pentaerythritol oder Dipentaerythritol.

Besonders bevorzugt sind (Meth)acrylester von alkoxylierten Mehrfachalkoholen wie beispielsweise ethoxyliertes Glyceroltriacrylat, Propoxyliertes Glyceroltriacrylat, ethoxyliertes Trimethylolpropan-trimethacrylat, ethoxyliertes Trimethylolpropan-triacrylat, propoxyliertes Trimethylolpropan-trimethacrylat, propoxyliertes Trimethylolpropantriacrylat, ethoxyliertes Pentaerythritol-trimethacrylat, ethoxyliertes Pentaerythritol-triacrylat, ethoxyliertes Pentaerythritol-tetramethacrylat, ethoxyliertes Pentaerythritol-tetraacrylat, ethoxyliertes Di-Pentaerythritol-trimethacrylat, ethoxyliertes Di-Pentaerythritol-tetramethacrylat, ethoxyliertes Di-Pentaerythritolpentamethacrylat, ethoxyliertes Di-Pentaerythritol-hexamethacrylat, ethoxyliertes Di-Pentaerythritol-triacrylat, ethoxyliertes Di-Pentaerythritol-tetraacrylat, ethoxyliertes Di-Pentaerythritol-pentaacrylat, ethoxyliertes Di-Pentaerythritol-hexaacrylat, propoxyliertes Pentaerythritol-trimethacrylat, propoxyliertes Pentaerythritol-triacrylat, propoxyliertes Pentaerythritol-tetramethacrylat, propoxyliertes Pentaerythritol-tetraacrylat, propxyliertes Di-Pentaerythritol-trimethacrylat, propoxyliertes Di-Pentaerythritol-tetramethacrylat, propoxyliertes Di-Pentaerythritolpentamethacrylat, propoxyliertes Di-Pentaerythritolhexamethacrylat, propoxyliertes Di-Pentaerythritol-triacrylat, propoxyliertes Di-Pentaerythritol-tetraacrylat, propxyliertes Di-Pentaerythritol-pentaacrylat, propoxyliertes Di-Pentaerythritol-hexaacrylat.

Solche an die Alkohole angebunden Alkoxygruppen stellen (Molekül)Kettenverlängerer dar. Die Kettenverlängerung kann vorzugsweise durch Ethoxylierung oder Propoxylierung erreicht werden. Für die Kettenverlängerung kommen weitere Verknüpfungsmöglichkeiten bspw. Etherbindungen, Esterbindungen, Amidbindungen, Urethanbindungen u.ä. in Frage, an die sich bevorzugt wiederum Ethylenglykol- oder Propylenglykolgruppen anschließen können.

Bevorzugte Kettenverlängerer sind bspw.

-CH$_2$-CH$_2$-

-CH$_2$-CH$_2$-CH$_2$-

-CH$_2$-CH$_2$-O-CH$_2$-CH$_2$-

-CH$_2$-CH$_2$-CH$_2$-O-CH$_2$-CH$_2$-CH$_2$-

u.s.w.

-O-CH$_2$-CH$_2$-

-O-CH$_2$-CH$_2$-CH$_2$-

-O-CH$_2$-CH$_2$-O-CH$_2$-CH$_2$-

-O-CH$_2$-CH$_2$-CH$_2$-O-CH$_2$-CH$_2$-CH$_2$-

u.s.w.

-O-CO- CH$_2$-CH$_2$-

-O-CO -CH$_2$-CH$_2$-CH$_2$-

-O-CO -CH$_2$-CH$_2$-O-CH$_2$-CH$_2$-

-O-CO -CH$_2$-CH$_2$-CH$_2$-O-CH$_2$-CH$_2$-CH$_2$-

u.s.w.

-CO- CH$_2$-CH$_2$-

-CO -CH$_2$-CH$_2$-CH$_2$-

-CO -CH$_2$-CH$_2$-O-CH$_2$-CH$_2$-

-CO -CH$_2$-CH$_2$-CH$_2$-O-CH$_2$-CH$_2$-CH$_2$-

u.s.w.

-NR$^3$-CO-CH$_2$-CH$_2$-

-NR$^3$-CO-CH$_2$-CH$_2$-CH$_2$-

-NR$^3$-CO-CH$_2$-CH$_2$-O-CH$_2$-CH$_2$-

-NR$^3$-CO-CH$_2$-CH$_2$-CH$_2$-O-CH$_2$-CH$_2$-CH$_2$-

u.s.w.

-O-CO-NR$^3$-CH$_2$-CH$_2$-

-O-CO-NR$^3$-CH$_2$-CH$_2$-CH$_2$-

-O-CO-NR$^3$-CH$_2$-CH$_2$-O-CH$_2$-CH$_2$-

-O-CO-NR$^3$-CH$_2$-CH$_2$-CH$_2$-O-CH$_2$-CH$_2$-CH$_2$-

u.s.w.

$$-NR^3-CO-O-CH_2-CH_2-$$

$$-NR^3-CO-O-CH_2-CH_2-CH_2-$$

$$-NR^3-CO-O-CH_2-CH_2-O-CH_2-CH_2-$$

$$-NR^3-CO-O-CH_2-CH_2-CH_2-O-CH_2-CH_2-CH_2-$$

u.s.w.

$$-NR^3-CO-NR^3-CH_2-CH_2-$$

$$-NR^3-CO-NR^3-CH_2-CH_2-CH_2-$$

$$-NR^3-CO-NR^3-CH_2-CH_2-O-CH_2-CH_2-$$

$$-NR^3-CO-NR^3-CH_2-CH_2-CH_2-O-CH_2-CH_2-CH_2-$$

u.s.w.

wobei $R^3$ H oder eine Alkyl-, bevorzugt eine Methylgruppe ist.

Die kettenverlängernde Gruppe ist bevorzugt endständig mit den vernetzenden Gruppen, bevorzugt einer Methacrylat-, einer Acrylatgruppe, Methacrylamid- oder Acrylamidgruppe funktionalisiert.

Als Vernetzungspunkt wird die Position der vernetzenden polymerisierbaren Gruppe bspw. die Position einer C=C-Doppelbindung im Monomer angesehen.

Die Kettenlänge ist bevorzugt derart, dass der Abstand der Vernetzungspunkte mindestens 7, bevorzugt mindestens 9, weiter bevorzugt 10 bis 30, besonders bevorzugt 11 bis 21 Bindungslängen beträgt. Der Abstand beträgt bevorzugt weniger als 50 Bindungslängen.

Mit Abstand der Vernetzungspunkte, ist der kürzeste Abstand der vernetzenden Gruppen bspw. zweier C=C-Doppelbindungen entlang des Moleküls zu verstehen. Es ist also nur die Konstitution des Moleküls gemeint, nicht etwa die wirkliche räumliche Lage der Gruppen zueinander, etwa durch Konfiguration oder Konformation bedingt.

Unter Bindungslänge ist der Abstand zweier Atome im Molekül zu verstehen, egal welche Art kovalente Bindung vorliegt und welche exakte Bindungslänge die einzelne kovalente Bindung aufweist.

Der Anteil der vernetzenden Monomere mit mindestens drei polymerisierbaren Gruppen und einem Abstand der Vernetzungspunkte von weniger als 10 Bindungslängen, bezogen auf die Gesamtmasse der Monomere, beträgt vorzugsweise weniger als 20 Gew.-%, weiter vorzugsweise weniger als 10 Gew.-%, weiter vorzugsweise weniger als 5 Gew.-%.

Durch die Einstellung eines Abstandes zwischen den vernetzenden Gruppen, wie oben definiert, und damit einhergehend die Einstellung einer entsprechenden Netzbogenlänge in vernetzten Polymeren können die Spannungen im ausgehärteten Polymer vermindert werden. Diese Verminderung der Spannungen führt dazu, dass es auch unter Temperaturwechselbelastungen wie beispielsweise einem als Testverfahren verwendeten Thermocycling zwischen 5 und 55°C nicht zu spannungsinduzierten Rissbildungen im Polymer kommt. Die mechanischen Eigenschaften und insbesondere die Dauerbeständigkeit des ausgehärteten Infiltranten werden so verbessert.

Der bevorzugte Anteil dieser Monomeren hängt von der Anzahl der vernetzenden Gruppen in der Monomermischung, der Größe der kettenverlängernden Gruppen und dem resultierenden PK ab.

Der erfindungsgemäße Infiltrant kann zusätzlich Monomere mit einer polymerisierbaren Gruppe enthalten. Diese können bevorzugt ausgewählt sein aus der Gruppe bestehend aus MMA, Methylmethacrylat; EMA, Ethylmethacrylat; n-BMA, n-Butylmethacrylat; IBMA, Isobutylmethacrylat, t-BMA, tert-Butylmethacrylat; EHMA, 2-Ethylhexylmethacrylat; LMA, Laurylmethacrylat; TDMA, Tridecylmethacrylat; SMA, Stearylmethacrylat; CHMA, Cyclohexylmethacrylat; BZMA, Benzylmethacrylat; IBXMA, Isobornylmethacrylate; HEMA, 2-Hydroxyethylmethacrylat; HPMA, 2-Hydroxypropylmethacrylat; DMMA, Dimethylaminoethylmethacrylat; DEMA, Diethylaminoethylmethacrylat; GMA, Glycidylmethacrylat; THFMA, Tetrahydrofurfurylmethacrylat; AMA, Allylmethacrylat; ETMA, Ethoxyethylmethacrylat; 3FM, Trifluorethylmethacrylat; 8FM, Octafluorpentylmethacrylat; AIB, Isobutylacrylat; TBA, tert-Butylacrylat; LA, Laurylacrylat; CEA, Cetylacrylat; STA, Stearylacrylat; CHA, Cyclohexylacrylat; BZA, Benzylacrylat; IBXA, Isobornylacrylat; 2-MTA, 2-Methoxyethylacrylat; ETA, 2-Ethoxyethylacrylat; EETA, Ethoxyethoxyethylacrylat; PEA, 2-Phenoxyethylacrylat; THFA, Tetrahydrofurfurylacrylat; HEA, 2-Hydroxyethylacrylat; HPA, 2-Hydroxypropylacrylat; 4HBA, 4-Hydroxybutylacrylat; DMA, Dimethylaminoethylacrylat; 3F, Trifluorethylacrylat; 17F, Heptadecafluorodecylacrylat; 2-PEA, 2-Phenoxyethylacrylat; TBCH, 4-tert-butylcyclohexylacrylat; DCPA, Dihydrodicyclopentadienylacrylat; EHA, 2-Ethylhexylacrylat; und 3EGMA, Triethylenglycolmonomethacrylat.

Die Monomere, Monomermischungen und/oder Infiltranten haben bevorzugt eine Dynamische Viskosität kleiner 50 mPas, weiter bevorzugt kleiner 30 mPas, besonders bevorzugt kleiner 20 mPas.

Die Mischung unterschiedlicher Monomeren dient insbesondere der Abstimmung der mechanischen Eigenschaften wie Härte und Festigkeit, der Durchhärtetiefe bzw. des Polymerisationsgrades, des Restmonomergehaltes, der Schmierschichtausprägung, des Schrumpfs, der Stabilität, der Wasseraufnahme sowie insbesondere der Spannungsfreiheit unter Erhalt einer hohen Penetrationsfähigkeit (PK > 100).

Insbesondere ist auch die Wasserverträglichkeit der Monomeren von Bedeutung etwa für den Fall, dass die Schmelzläsion nach der Vorbereitung (Ätzen, Spülen, Trocknen) noch Restfeuchtigkeit aufweist. Bestimmte Monomere können Restfeuchtigkeit aufnehmen und so die Penetration weiter verbessern. Hierfür eignen sich besonders wasserlösliche und/oder phasenvermittelnde Ester der (Meth)acrylsäure, bspw. HEMA, 2-Hydroxyethylmethacrylat oder GDMA, Glycerindimethacrylat oder GMA, Glycerinmonomethacrylat.

Die Mischung mit weiteren Monomeren kann weiterhin insbesondere der Abstimmung weiterer vorteilhafter Eigenschaften wie hohe Oberflächenglätte (plaqueverhindernd), Fluoridfreisetzung, Röntgenopazität, Haftung am Schmelz, Langzeitfarbstabilität, Biokompatibilität u.s.w. dienen.

Der Infiltrant kann auch dem Fachmann bspw. aus WO 02/062901, WO 2006/031972 oder EP 1714633 bekannte und im Dentalbereich gebräuchliche hyperverzweigte Monomere, bspw. Dendrimere aufweisen, insbesondere um den Restmonomergehalt zu senken und die Biokompatibilität zu verbessern.

Der Infiltrant kann dem Fachmann bspw. aus EP 1285947 oder EP 1849450 bekannte und im Dentalbereich gebräuchliche bakterizide Monomere enthalten.

Die Monomermischungen bzw. der Infiltrant haben dabei einen PK > 100, besonders bevorzugt > 200.

Der Infiltrant enthält Mittel zur Härtung des Infiltranten. Die Mittel zur Härtung können dem Fachmann bekannte und im Dentalbereich gebräuchliche Initiatoren insbesondere lichtaktivierte Initiatorsysteme aber auch chemisch aktivierende Initiatoren sein oder Mischungen der verschiedenen Systeme.

Die hier verwendbaren Initiatoren können z.B. Photoinitiatoren sein. Diese sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm und optional durch die zusätzliche Reaktion mit einem oder mehreren Coinitiatoren die Aushärtung des Materials bewirken können. Bevorzugt werden hier Phosphinoxide, Bezoinether, Benzilketale, Acetophenone, Benzophenone, Thioxanthone, Bisimidazole, Metallocene, Fluorone, α-Dicarbonylverbindungen, Aryldiazoniumsalze, Arylsulfoniumsalze, Aryliodoniumsalze, Ferroceniumsalze, Phenylphosphoniumsalze oder eine Mischung aus diesen Verbindungen eingesetzt.

Besonders bevorzugt werden Diphenyl-2,4,6-trimethylbenzoyl-phosphinoxid, Benzoin, Benzoinalkylether, Benzildialkylketale, α-Hydroxy-acetophenon, Dialkoxyacetophenone, α-Aminoacetophenone, i-Propylthioxanthon, Campherchinon, Phenylpropandion, 5,7-Diiodo-3-butoxy-6-fluoron, (eta-6-Cumol)(eta-5-cyclopentadienyl)eisen-hexafluorophosphat, (eta-6-Cumol)(eta-5-cyclopentadienyl)eisentetrafluoroborat, (eta-6-Cumol)(eta-5-cyclopentadienyl)eisen-hexafluoroantimonat, substituierte Diaryliodoniumsalze, Triarylsulfoniumsalze oder eine Mischung aus diesen Verbindungen eingesetzt.

Als Coinitiatoren für eine photochemische Aushärtung werden bevorzugt tertiäre Amine, Borate, organische Phosphite, Diaryliodoniumverbindungen, Thioxanthone, Xanthene, Fluorene, Fluorone, α-Dicarbonylverbindungen, kondensierte Polyaromaten oder eine Mischung aus diesen Verbindungen eingesetzt. Besonders bevorzugt werden N,N-Dimethyl-p-toluolidin, N,N-Dialkyl-alkyl-aniline, N,N-Dihydroxyethyl-p-toluidin, 2-Ethylhexyl-p-(dimethyl-amino)-benzoat, Butyrylcholin-triphenylbutyl-borat, N,N-Dimethyl-4-tert.-butylanilin, N,N-Diethyl-3,5-ditert.-butylanilin, 4-tert..-butylanilin oder eine Mischung aus diesen Verbindungen eingesetzt.

Zu 100 Teilen Harz bzw. Harzmischung werden bevorzugt 0,05 bis 2 Teile Photoinitiator zugegeben, weiter bevorzugt 0,1 bis 1 Teile. Der Coinitiator ist im Überschuss, 1 - 5fach, bevorzugt 1 - 3fach enthalten, bezogen auf den Initiator.

Der Infiltrant kann aus einem Kit mit wenigstens zwei Komponenten hergestellt werden. Zwei Komponenten enthaltende Infiltranten haben den Vorteil, dass sie selbsthärtend (chemische Aushärtung) gestaltet werden können. In einer Ausführungsform enthält eine erste Komponente Monomere und chemisch aktivierbare Initiatoren und eine zweite Komponente geeignete Aktivatoren.

Für eine chemische Aushärtung bei Raumtemperatur wird i. a. ein Redoxinitiatorsystem verwendet, das aus einem bzw. mehreren Initiatoren und einem als Aktivator dienenden Coinitiator bzw. Coinitiatoren besteht. Aus Gründen der Lagerstabilität werden Initiator bzw. Initiatoren und Coinitiator bzw. Coinitiatoren in räumlich voneinander getrennten Teilen des erfindungsgemäßen Infiltranten eingearbeitet, d.h. es liegt ein mehrkomponentiges, bevorzugt ein zweikomponentiges Material vor. Als Initiator bzw. Initiatoren werden bevorzugt anorganische und/oder organische Peroxide, anorganische und/oder organische Hydroperoxide, Barbitursäurederivate, Malonylsulfamide, Protonensäuren, Lewis- oder Broensted-Säuren bzw. Verbindungen, die solche Säuren freisetzen, Carbeniumionen-Donatoren wie z. B. Methyltriflat oder Triethylperchlorat oder eine Mischung aus diesen Verbindungen und als Coinitiator bzw. als Coinitiatoren bevorzugt tertiäre Amine, Schwermetallverbindungen, insbesondere Verbindungen der 8. und der 9. Gruppe des Periodensystems ("Eisen- und Kupfergruppe"), Verbindungen mit ionogen gebundenen Halogenen oder Pseudohalogenen wie z. B. quartäre Ammoniumhalogenide, schwache Broenstedt-Säuren wie z. B. Alkohole und Wasser oder eine Mischung aus diesen Verbindungen eingesetzt.

In einer besonders einfachen Ausführungsform ist das Gerät zum Auftragen des Infiltranten (Applikationshilfe) auf den Zahn mit dem Aktivator getränkt oder belegt.

In einer weiteren Ausführungsform enthält eine erste Komponente Monomere und als Initiatoren Salze CH-acider Verbindungen wie Barbitursäurederivate und eine zweite Komponente Monomere und eine aktivierende Komponente, bevorzugt eine stärker acide Säure als die CH-acide Verbindung.

In einer besonders einfachen Ausführungsform enthält ein Gerät zum Auftragen des Infiltranten (Applikationshilfe) auf den Zahn eine Mischkammer und/oder Mischelemente enthaltende Kanülen.

Der Infiltrant kann Stabilisatoren enthalten. Bevorzugt werden UV-Stabilisatoren. Geeignete UV-Stabilisatoren sind dem Fachmann bekannt beispielhaft sei hier Chimasorb® und Tinuvin® (Ciba) genannt.

Der Infiltrant kann (nicht polymerisierbare) Lösungsmittel enthalten. Bevorzugt sind dann mittelflüchtige Lösungsmittel wie bspw. Alkohole, höhermolekulare Ketone und Ether sowie Ester, u.s.w. Besonders bevorzugt sind Alkohole, insbesondere Ethanol oder Lösungsmittel mit ähnlichem Verdampfungsverhalten.

Der Infiltrant enthält 20 Masse-% oder weniger, bevorzugt weniger als ungefähr 10 Masse-%, besonders bevorzugt kein Lösungsmittel.

Der Infiltrant kann mindestens einen fluoreszierenden Farbstoff und/oder Farbpigmente enthalten, um das Erscheinungsbild zu verbessern bzw. dem Zahnschmelz anzupassen. Geeignete fluoreszierende Farbmittel sind dem Fachmann bekannt und bspw. in der US 2004/017928 A1 beschrieben. Der Infiltrant kann sonstige Farbmittel insbesondere zur Herstellung verschiedener Zahnfarben enthalten. Der Infiltrant kann die Farbe wechselnde Farbstoffe enthalten, die die infiltrierte Läsion anzeigen und durch Farbumschlag die Aushärtung des Infiltranten anzeigen. Bevorzugt wird der Farbstoff nach Aushärtung des Infiltranten farblos. Der Farbstoff kann radikalisch reaktiv sein.

Der Farbumschlag kann auch von anderen Einflüssen abhängen bspw. vom pH-Wert.

Der Farbstoff kann adsorptive Eigenschaften, insbesondere bezüglich des Zahnschmelzes aufweisen, so dass er sich in der oberen Schicht der Läsion anreichert. Der Farbumschlag kann dann auch im Zwischenzahnbereich besser gesehen werden.

Der Farbstoff kann nicht-adsorptive Eigenschaften aufweisen und tief in die Läsion penetrieren, so dass bspw. die Durchdringung besser kontrolliert werden kann.

Der Infiltrant kann thermo- und/oder photochrome Zusätze enthalten, durch die der infiltrierte Bereich bei Bestrahlung mit entsprechendem Licht bzw. Temperaturänderung angezeigt wird.

Der Infiltrant kann Kontrastmittel zur Erreichung von Röntgenkapazität, bevorzugt organische Verbindungen, wie sie in der Europäischen Patentanmeldung EP 08014437 offenbart sind, enthalten.

Gegenstand der Erfindung ist der Infiltrant zur Anwendung in der Behandlung und/oder Prävention von kariösen Schmelzläsionen. Eine solche Verwendung kann die nachfolgenden Schritte umfassen:

1. Entfernen einer dünnen Oberflächenschicht der Schmelzläsion durch Ätzmittel
2. Abspülen des Ätzmittels
3. Trocknen der Läsion mit einem Trocknungsmittel
4. Infiltration der Läsion mit einem Infiltranten
5. Entfernen von Überschüssen (optional)
6. Härten des Infiltranten
7. Infiltration der Läsion mit einem Infiltrant (optional)
8. Entfernen von Überschüssen (optional)
9. Härten des Infiltranten (optional)
10. Politur der infiltrierten Läsionsoberfläche (optional)

Bei einzelnen Schritten der Infiltrationsmethode kann das gewünschte Ergebnis durch Anwendung von Schall und/oder Ultraschall weiter verbessert werden.

Bevorzugte Ätzmittel sind Gele starker Säuren wie Salzsäure.

Bevorzugte Trocknungsmittel sind toxikologisch unbedenkliche Lösungsmittel mit hohem Dampfdruck. Besonders bevorzugt sind Trocknungsmittel mit einem Dampfdruck größer als der des Wassers (23 mbar [20°C]). Diese sind bspw. ausgewählt aus Alkoholen, Ketonen, Ethern, Estern usw. Besonders bevorzugt ist Ethanol.

Das Trocknungsmittel kann Bestandteile des Initiatorsystems enthalten, die nach dessen Verdunstung in der Läsion verbleiben.

Das Trocknungsmittel kann einen Filmbildner enthalten.

Die Erfindung umfasst weiterhin einen Kit zur Durchführung des Infiltrationsverfahrens. Dieser umfasst

1. Ätzmittel
2. Trocknungsmittel (optional)
3. Infiltrant.

Im Folgenden ist die Erfindung anhand einiger Beispiele erläutert.

Monomermischungen wurden hergestellt und deren Penetrationskoeffizient (PK), Durchhärtetiefe, mechanisches Verhalten und Haftfestigkeit (shear bond strength) überprüft.

Die folgenden Komponenten kamen zum Einsatz:

| TEDMA | Triethlenglycoldimethacrylat |
|---|---|
| E3TMPTA | Trimethylolpropan ethoxyliert mit durchschn. 1 EO-Einheiten pro Methylolgruppe und terminal acryliert |
| MDP | 10-Methacryloyloxydecyldihydrogenphosphat |
| CQ | Campherchinon |
| EHA | Ethylhexyl-p-N,N-dimethylaminobenzoat |
| BHT | 2,6-Di-tert.-butylphenol |

Prüfungsmethoden

*Oberflächenspannung*

Die Oberflächenspannung der Harze wurde mittels Konturanalyse eines hängenden Tropfens durchgeführt (DSA 10, KRÜSS GmbH). Die Messung der Oberflächenspannung wurde an neu gebildeten Tropfen über einen Zeitraum von 30 s durchgeführt, wobei etwa alle 5 s ein Messwert aufgenommen wurde. Hierzu wurden die Harze mit einer feinen Spritze ausgebracht und der sich bildende Tropfen mit einer Digitalkamera gefilmt. Aus der charakteristischen Form und Größe des Tropfens wurde die Oberflächenspannung gemäß der Young-Laplace-Gleichung bestimmt. Für jedes Harz wurden so 3 Messungen durchgeführt, deren Mittelwert als Oberflächenspannung angegeben wurde.

Kontaktwinkel

Für jede Einzelmessung wurde Schmelz aus Rinderzähnen verwendet. Hierzu wurden Rinderzähne in einem Kunstharz eingebettet und die Schmelzfläche mittels Schleifmaschine (Struers GmbH) mit Schleifpapieren (80, 500 und 1200 Körnung) nass poliert, so dass plane etwa 0,5x1,0 cm große Schmelzflächen für die Kontaktwinkel-Messungen zur Verfügung standen. Die Schmelzproben wurden bis zur Messung in destilliertem Wasser gelagert und vor der Messung mit Ethanol und Druckluft getrocknet.

Die Messung des Kontaktwinkels erfolgte mit Hilfe eines Video-Kontaktwinkelmessgerät (DSA 10, KRÜSS GmbH). Hierbei wurde mittels Mikroliterspritze ein Tropfen der Harzmischung auf die Schmelzfläche gebracht, innerhalb von 10 s bis zu 40 Einzelaufnahmen des Tropfens rechnergesteuert aufgenommen und per Tropfenkonturanalyse-Software der Kontaktwinkel ermittelt.

*Durchhärtetiefe*

Die Durchhärtetiefen der Harze wurden entsprechend der Prüfung "Polymerisationstiefe" nach ISO 4049:2000 bestimmt. Hierzu wurden die Harze in zylindrische Teflonformen (5 mm im Durchmesser, 10 mm hoch) gefüllt und mit einer Halogenlampe (Translux EC, Heraeus Kulzer GmbH) 15 oder 20 s von oben belichtet. Direkt nach der Belichtung wurden die ausgehärteten Probekörper entformt und mit einem Plastikspatel von unausgehärtetem Material befreit. Die Höhe des ausgehärteten Zylinderkegels wurde als Durchhärtetiefe (DHT) angegeben.

*Dynamische Viskosität*

Die Viskosität der Harze wurde bei 23°C mittels dynamischen Platte-Platte-Viskosimeter (Dynamic Stress Rheometer, Rheometric Scientific Inc.) gemessen. Es wurde im "Steady Stress Sweep"-Modus bei Spaltgrößen von 0,1 bis 0,5 mm im Bereich von 0 bis 50 Pa Schubspannung ohne Vorscherung der Harze gemessen.

*Shear Bond Strength (SBS) Messungen*

Zur Messung der Haftung der erfindungsgemäßen Infiltranten wurden Shear-Bond-Strength-(SBS)-Versuche durchgeführt. Hierzu wurden Rinder-Schneidezähne, aus denen zuvor die Pulpa entfernt wurde und die nachträglich in 0,5 Gew% Chloramin-T Lösung in Wasser gelagert wurden, auf der Vorderseite naß plan geschliffen. Je nach Versuchsbedingungen wurden die planen Schmelzoberflächen mit einem 15%igen HCl-Gel durch das Einwirken für 2 Minuten mikroabradiert oder unbehandelt gelassen. Anschließend wurden die erfindungsgemäßen Infiltranten oder die Referenzbeispiele mittels Mirobrush auf die geätzte Schmelzfläche aufgebracht und 240s einwirken gelassen. Anschließend wurde der infiltrierte Bereich 40s mit einer Halogenlampe [Translux EC, Firma Firma Heraeus Kulzer] belichtet. Eine zweiteilige Teflonform mit einer Kavität von 3,0 mm Durchmesser wurde aufgesetzt und das lichthärtende Dentalkomposit EcuSphere Shape (Feinstglas-Hybrid-Komposit; DMG) hineingefüllt und 40s mit einer Halogenlampe [Translux EC, Firma Firma Heraeus Kulzer] belichtet. Die Teflonform wurde jeweils entfernt und die Verklebung in Wasser zunächst 23h bei 37°C, dann 1h bei 23°C gelagert. Die Verklebung wurde anschließend in eine Halterung zur Messung der SBS eingespannt und in einer Apparatur zur Bestimmung eines Kraft-Weg-Diagramms (Z010, Zwick GmbH, Ulm) bei einem Vorschub von 0,5mm/min gemäß ISO/TS 11405:2003 "Dental materials - Testing of adhesion to tooth structure" vermessen. Die Ergebnisse wurden in Form eines Mittelwertes, einer Standardabweichung (Ausdruck in Klammern) und der Summe der Einzelmessungen (n=6-8) notiert.

Beschreibung der Herstellung von Beispielen und Referenzbeispielen.

Die Harze wurden gemäß Tabelle durch Verrühren der entsprechenden Komponenten hergestellt. Für die Untersuchungen Durchhärtetiefe und Haftfestigkeit (shear bond strength) wurden zu den Harzmischungen 0,45 bzw. 0,5 Gew.-% CQ, 0,76 bzw. 0,84 Gew.-% EHA und 0,002 Gew.-% BHT zugesetzt. Alle Mischungen wurden bis zur optisch klaren Lösung gerührt.

| | Referenz 1 | Beispiel 1 | Beispiel 2 | Referenz 2 | Beispiel 3 | Beispiel 4 |
|---|---|---|---|---|---|---|
| TEDMA | 100 | 99 | 90 | 80 | 79,2 | 72 |
| E3TMPTA | | | | 20 | 19,8 | 18 |
| MDP | | 1 | 10 | | 1 | 10 |
| CQ | 0,5 | 0,5 | 0,45 | 0,5 | 0,5 | 0,45 |
| EHA | 0,84 | 0,84 | 0,76 | 0,84 | 0,84 | 0,76 |
| BHT | 0,002 | 0,002 | 0,002 | 0,002 | 0,002 | 0,002 |
| Viskosität [mPas] | 10 | 8 | 11,9 | 12 | 12,0 | 17,0 |
| Oberflächenspannung [mN/m] | 35,07 | 35,37 | 35,12 | 35,37 | 35,96 | 35,63 |
| Kontaktwinkel Schmelz [°] | 0,4 | 0,8 | 1,7 | 2,4 | 1,0 | 2.1 |
| Penetrationskoeffizient [cm/s] | **175** | **221** | **148** | **147** | **150** | **105** |
| DHT [mm] nach 20s Belichtung | **0** | **10** | **10** | **4,1** | **10** | **10** |
| DHT [mm] nach 15s Belichtung | **0** | **6,4** | **7,5** | **3,5** | **8,04** | **9,5** |
| SBS-Messung [MPa] auf ungeätztem Rinderschmelz; Mittelwert(SD); Zahl der Einzelmessungen | **3,8** (0,8) n=8 | **10,1**(1,8) n=8 | | | **12,1**(1,6) n=7 | **14,7**(1,9) n=6 |
| SBS-Messung [MPa] auf HCl-geätztem Rinderschmelz; Mittelwert (SD); Zahl der Einzelmessungen | **21,0** (1,4) n=8 | **22,7**(2,4) n=7 | | | **24,8**(2,3) n=8 | **26,5**(4,1) n=6 |

Die Zusammensetzung in dieser Tabelle ist in Gewichtsteilen angegeben.

Die Tabelle zeigt, dass der Zusatz auch geringer Mengen des sauren Monomers MDP zu einer deutlich verbesserten Durchhärtetiefe führt. Die Kombination des Zusatzes eines sauren Monomers MDP und eines vernetzenden Monomers mit drei polymerisierbaren Gruppen (E3TMPTA) führt zu ausgezeichneten Durchhärtetiefen auch bei einer geringen Belichtungsdauer.

Ferner zeigt die Tabelle, dass die erfindungsgemäßen Beispiele eine verbesserte Haftung auf mit HCl geätztem Rinderschmelz zeigen. Ein besonderer Vorteil ist die drastisch verbesserte Haftung auf ungeätztem Rinderschmelz.

**Patentansprüche**

1. Infiltrant für die Dentalapplikation, der vernetzende Monomere und 20 Gew.-% oder weniger Lösungsmittel enthält, **dadurch gekennzeichnet, dass** der Anteil vernetzender Monomere mit zwei polymerisierbaren Gruppen 99,8 bis 50 Gew.-%, beträgt, und dass er einen Penetrationskoeffizienten PK > 100 cm/s aufweist und 0,05 - 20 Gew.-% säuregruppenhaltige Monomere enthält, wobei die Säuregruppen ausgewählt sind aus der Gruppe bestehend aus Carbon-, Sulfon-, Phosphor- und Phosphonsäuregruppen, und wobei der Penetrationskoeffizient wie folgt definiert ist:

$$PK = \left( \frac{\gamma . \cos\theta}{2\eta} \right)$$

mit:

γ Oberflächenspannung des flüssigen Harzes (gegen Luft),
θ Kontaktwinkel des flüssigen Harzes (gegen Zahnschmelz),
η dynamische Viskosität des flüssigen Harzes;

zur Anwendung in der Behandlung und/oder Prävention von kariösen Schmelzläsionen.

2. Infiltrant nach Anspruch 1 zur Anwendung in der Behandlung und/oder Prävention von kariösen Schmelzläsionen, **dadurch gekennzeichnet, dass** der Gehalt an säuregruppenhaltigen Monomeren 0,1 bis 15 Gew.-%, vorzugsweise 0,2 bis 10 Gew.-%, weiter vorzugsweise 0,5 bis 5 Gew.-%, weiter vorzugsweise 0,5 bis 2 Gew.-% beträgt.

3. Infiltrant nach Anspruch 1 oder 2 zur Anwendung in der Behandlung und/oder Prävention von kariösen Schmelzläsionen, **dadurch, gekennzeichnet, dass** die säuregruppenhaltigen Monomere eine oder zwei, vorzugsweise eine polymerisierbare Gruppe enthalten.

4. Infiltrant nach einem der Ansprüche 1 bis 3 zur Anwendung in der Behandlung und/oder Prävention vorn kariösen Schmelzläsionen, **dadurch gekennzeichnet, dass** die säuregruppenhaltigen Monomere Acrylate, Methacrylate, Acrylamide und/oder Methylacrylamide sind.

5. Infiltrant nach einem der Ansprüche 1 bis 4 zur Anwendung in der Behandlung und/oder Prävention von kariösen Schmelzläsionen, **dadurch gekennzeichnet, dass** der Anteil vernetzender Monomere mit zwei polymerisierbaren Gruppen 99,5 bis 60 Gew.-%, vorzugsweise 99 bis 60 Gew.-%, weiter vorzugsweise 99 bis 70 Gew.-% beträgt.

6. Infiltrant nach einem der Ansprüche 1 bis 5 zur Anwendung in der Behandlung und/oder Prävention von kariösen Schmelzläsionen, **dadurch gekennzeichnet, dass** die vernetzenden Monomere mit zwei polymerisierbaren Gruppen Ester der Acryl- oder Meth
acrylsäure sind und vorzugsweise ausgewählt sind aus der Gruppe bestehend aus Allylmethacrylat; Allylacrylat; PRDMA, 1,3-Propandioldimethacrylat; BDMA, 1,3-Butandioldimethacrylat; BDDMA, 1,4-Butandioldimethacrylat; PDDMA, 1,5-Pentandioldimethacrylat; NPGDMA, Neopentylglykoldimethacrylat; HDDMA, 1,6-Hexandioldimethacrylat; NDDMA, 1,9-Nonandioldimethacrylat; DDDMA, 1,10-Decandioldimethacrylat; DDDDMA, 1,12-Dodecandioldimethacrylat; PRDA, 1,3-Propandioldiacrylat; BDA, 1,3-Butandioldiacrylat; BDDA, 1,4-Butandioldiacrylat; PDDA, 1,5-Pentandioldiacrylat; NPGDA, Neopentylglykoldiacrylat; HDDA, 1,6-Hexandioldiacrylat; NDDA, 1,9-Nonandioldiacrylat; DDDA, 1,10-Decandioldiacrylat; DDDDA, 1,12-Dodecandioldimethacrylät; EGDMA, Ethylenglykoldimethacrylat; DEGDMA, Diethylenglykoldimethacrylat; TEDMA, Triethylenglykoldimethacrylat; TEGDMA, Tetraethylenglykoldimethacrylat; EGDA, Ethylenglykoldiacrylat; DEGDA, Diethylenglykoldiacrylat; TEDA, Triethylenglykoldiacrylat; TEGDA, Tetraethylenglykoldiacrylat; PEG200DMA, Polyethylenglykol 200 Dimethacrylat; PEG300DMA, Polyethylenglykol 300 Dimethacrylat; PEG400DMA, Polyethylenglykol 400 Dimethacrylat; PEG600DMA, Polyethylenglykol 600 Dimethacrylat; PEG200DA, Polyethylenglykol 200 Diacrylat; PEG300DA, Polyethylenglykol 300 Diacrylat; PEG400DA, Polyethylenglykol 400 Diacrylat; PEG600DA, Polyethylenglykol 600 Diacrylat; PPGDMA, Polypropylenglykoldimethacrylat; PPGDA, Polypropylenglykoldiacrylat; NPG (PO) 2 DMA, Propoxyliertes (2) Neopentylglykoldimethacrylat; NPG(PO)2DA, Propoxyliertes (2) Neopentylglykoldiacrylat; Bis-MA, Bisphenol-A-dimethacrylat; Bis-GMA, Bisphenol-A-glyceroldimethacrylat; BPA (EO) DMA, Ethoxyliertes Bisphenol-A-Dimethacrylat(EO=1-30); BPA (PO) DMA, Propoxyliertes Bisphenol-A-Dimethacrylat(PO=1-30); BPA(EO)DA, Ethoxyliertes Bisphenol-A-Diacrylat (EO=1-30); BPA(PO)DA, Propoxyliertes Bisphenol-A-Diacrylat (PO=1-30); BPA(PO)GDA, Propoxyliertes Bisphenol-A-glyceroldiacrylat; UDMA, Diurethandimethacrylat; TCDDMA, Tricyclo[5.2.1.0]decandimethanoldimethacrylate; TCDDA, Tricyclo[5.2.1.0]decandimethanoldiacrylate; EBA, N,N'-Ethylenbisacrylamid; DHEBA, N,N'-(1,2-Dihydroxyethylen)bisacrylamid; DEPBA, N,N'-Diethyl(1,3-propylen)bisacrylamid; TMHMBMA, N,N'-(2,2,4-Trimethylhexamethylen)bismethacrylamid; und Bis[2-(2methylacrylamino)ethoxycarbonyl]hexamethylendiamin.

**7.** Infiltrant nach einem der Ansprüche 1 bis 6 zur Anwendung in der Behandlung und/oder Prävention von kariösen Schmelzläsionen, **dadurch gekennzeichnet, dass** er zusätzlich Monomere mit mindestens drei polymerisierbaren Gruppen enthält, wobei der Anteil vernetzender Monomere mit mindestens drei polymerisierbaren Gruppen vorzugsweise 10 bis 50 Gew.-%, weiter vorzugsweise 10 bis 30 Gew.-%, beträgt, wobei die vernetzenden Monomere mit mindestens drei polymerisierbaren Gruppen vorzugsweise einen Abstand der Vernetzungspunkte von wenigstens 7 Bindungslängen, weiter vorzugsweise höchstens 50 Bindungslängen, weiter vorzugsweise 10 bis 30 Bindungslängen, weiter vorzugsweise 1'1 bis 21 Bindungslängen aufweisen, und wobei weiter vorzugsweise der Anteil der vernetzenden Monomere mit mindestens drei polymerisierbaren Gruppen und einem Abstand der Vernetzungspunkte von weniger als 10 Bindungslängen, bezogen auf die Gesamtmasse der Monomere, weniger als 20 Gew.-%, vorzugsweise weniger als 10 Gew.-%, weiter vorzugsweise weniger als 5 Gew.-% beträgt.

**8.** Infiltrant nach Anspruch 7 zur Anwendung in der Behandlung und/oder Prävention von kariösen Schmelzläsionen, **dadurch gekennzeichnet, dass** die vernetzenden Monomere mit mindestens drei polymerisierbaren Gruppen die nachfolgende Formel aufweisen,

$$R^1[X_kR^2{}_1Y_m]_n$$

mit folgenden Bedeutungen

$R^1$ ist ein linearer oder verzweigter Kohlenwasserstoff mit 3-24 C-Atomen, enthaltend Alkyl, Cycloalkyl oder Aryl; optional enthaltend O, N, Si, S, P als Heteroatome, beispielsweise Siloxan und/oder Cyclosiloxan und/oder Carbosilan und/oder Cyclocarbosilan und/oder insbesondere Ether- oder Polyethergruppen, Polyestergruppen, Polysiloxangruppen oder Polycarbosilangruppen; optional substituiert durch Hydroxyl und/oder Carbonyl und/oder Halogen (bevorzugt Fluor) und/oder Ammoniumalkylengruppen und/oder Siloxan und/oder Cyclosiloxan und/oder Carbosilan und/oder Cyclocarbosilan; $R^2$ ist ein linearer oder verzweigter Kohlenwasserstoff mit 1-16 C-Atomen, enthaltend Alkyl, Cycloalkyl oder Aryl; optional enthaltend O, N, Si, S, P als Heteroatome, beispielsweise Siloxan und/oder Cyclosiloxan und/oder Carbosilan und/oder Cyclocarbosilan und/oder insbesondere Ether- oder Polyethergruppen, Polyestergruppen, Polysiloxangruppen oder Polycarbosilangruppen; optional substituiert durch Hydroxyl und/oder Carbonyl und/oder Halogen (bevorzugt Fluor) und/oder Ammoniumalkylengruppen und/oder Siloxan und/oder Cyclosiloxan und/oder Carbosilan und/oder Cyclocarbosilan; X ist eine verknüpfende Gruppe gleich oder verschieden ausgewählt aus einer Ethergruppe, Carbonylgruppe, Estergruppe, Amidgruppe, Urethangruppe oder Harnstoffgruppe; Y ist eine Gruppe gleich oder verschieden enthaltend eine polymerisierbare Doppelbindung und/oder eine ringöffnend polymerisierbare Gruppe und/oder eine Thiolgruppe und/oder eine Epoxidgruppe und/oder eine Vinyl; bevorzugt (Meth)acrylat und/oder (Meth)acrylamid; k ist 0 oder 1; l ist 0 oder 1 m ist mindestens 1 n ist mindestens 1 $m \times n$ ist mindestens 3.

**9.** Infiltrant nach Anspruch 7 oder 8 zur Anwendung in der Behandlung und/oder Prävention von kariösen Schmelzläsionen, **dadurch gekennzeichnet, dass** die vernetzenden Monomere mit mindestens drei polymerisierbaren Gruppen ausgewählt sind aus der Gruppe bestehend aus TMPTMA, Trimethylolpropantrimethacrylat; TMPTA, Trimethylolpropantriacrylat; DTMPTA, Di-Trimethylolpropantetra(meth)acrylat; DIPENTA, Di-Pentaerythritolpenta(meth)acrylat; GPTA, propoxyliertes Glyceryltri(meth)acrylat; DPEHA, Dipentaerythritolhexa(meth)acrylat; und ethoxyliertes Trimethylolpropantri(meth)acrylat; vorzugsweise ausgewählt sind aus der Gruppe bestehend aus propoxyliertes Glyceryltri(meth)acrylat; ethoxyliertes Trimethylolpropantrimethacrylat, ethoxyliertes Trimethylolpropantriacrylat, propoxyliertes Trimethylolpropan-trimethacrylat, propoxyliertes Trimethylolpropan-triacrylat, ethoxyliertes Pentaerythritol-trimethacrylat, ethoxyliertes Pentaerythritol-triacrylat, ethoxyliertes Pentaerythritol-tetramethacrylat, ethoxyliertes Pentaerythritol-tetraacrylat, ethoxyliertes Di-Pentaerythritol-trimethacrylat, ethoxyliertes Di-Pentaerythritol-tetramethacrylat, ethoxyliertes Di-Pentaerythritolpentamethacrylat, ethoxyliertes Di-Pentaerythritolhexamethacrylat, ethoxyliertes Di-Pentaerythritol-triacrylat, ethoxyliertes Di-Pentaerythritol-tetraacrylat, ethoxyliertes Di-Pentaerythritol-pentaacrylat, ethoxyliertes Di-Pentaerythritol-hexaacrylat, propoxyliertes Pentaerythritol-trimethacrylat, propoxyliertes Pentaerythritol-triacrylat, propoxyliertes Pentaerythritol-tetramethacrylat, propoxyliertes Pentaerythritol-tetraacrylat, propxyliertes Di-Pentaerythritol-trimethacrylat, propoxyliertes Di-Pentaerythritol-tetramethacrylat, propoxyliertes Di-Pentaerythritol-pentamethacrylat, propoxyliertes Di-Pentaerythritol-hexamethacrylat, propoxyliertes Di-Pentaerythritol-triacrylat, propoxyliertes Di-Pentaerythritol-tetraacrylat, propxyliertes Di-Pentaerythritol-pentaacrylat, propoxyliertes Di-Pentaerythritol-hexaacrylat.

10. Infiltrant nach einem der Ansprüche 1 bis 9 zur Anwendung in der Behandlung und/oder Prävention von kariösen Schmelzläsionen, **dadurch gekennzeichnet, dass** er eine dynamische Viskosität von 50 mPas oder weniger, vorzugsweise 30 mPas oder weniger, weiter vorzugsweise 20 mPas oder weniger aufweist.

11. Infiltrant nach einem der Ansprüche 1 bis 10 zur Anwendung in der Behandlung und/oder Prävention von kariösen Schmelzläsionen, **dadurch gekennzeichnet, dass** er 10 Gew.-% oder weniger Lösungsmittel enthält, besonders bevorzugt im Wesentlichen kein Lösungsmittel enthält.

12. Kit zur Herstellung eines Infiltranten nach einem der Ansprüche 1 bis 11 zur Anwendung in der Behandlung und/oder Prävention von kariösen Schmelzläsionen, **dadurch gekennzeichnet, dass** der Kit eine erste Komponente mit Monomeren und chemisch aktivierbaren Initiatoren und eine zweite Komponente mit Aktivatoren enthält, wobei der Kit vorzugsweise zusätzlich Ätz- und/oder Trocknungsmittel aufweist.

## Claims

1. Infiltrant for dental application, which contains crosslinking monomers and 20 wt.% or less solvent, **characterised in that** the proportion of crosslinking monomers having two polymerisable groups is 99.8 to 50 % by weight and **in that** it has a penetration coefficient PK > 100 cm/s and contains 0.05-20 % by weight acid-group-containing monomers, the acid groups being selected from the group consisting of carboxylic acid groups, sulfonic acid groups, phosphoric acid groups and phosphonic acid groups, and the penetration coefficient being defined as follows:

$$PK = \left( \frac{\gamma . \cos\theta}{2\eta} \right)$$

where:

$\gamma$ is the surface tension of the liquid resin (against air),
$\theta$ is the contact angle of the liquid resin (against dental enamel),
$\eta$ is the dynamic viscosity of the liquid resin;

for use in the treatment and/or prevention of carious enamel lesions.

2. Infiltrant according to claim 1 for use in the treatment and/or prevention of carious enamel lesions, **characterised in that** the content of acid-group-containing monomers is 0.1 to 15 % by weight, preferably 0.2 to 10 % by weight, more preferably 0.5 to 5 % by weight, more preferably 0.5 to 2 % by weight.

3. Infiltrant according to either claim 1 or claim 2 for use in the treatment and/or prevention of carious enamel lesions, **characterised in that** the acid-group-containing monomers contain one or two, preferably one, polymerisable group(s).

4. Infiltrant according to any of claims 1 to 3 for use in the treatment and/or prevention of carious enamel lesions, **characterised in that** the acid-group-containing monomers are acrylates, methacrylates, acrylamides and/or methylacrylamides.

5. Infiltrant according to any of claims 1 to 4 for use in the treatment and/or prevention of carious enamel lesions, **characterised in that** the proportion of crosslinking monomers having two polymerisable groups is 99.5 to 60 % by weight, preferably 99 to 60 % by weight, more preferably 99 to 70 % by weight.

6. Infiltrant according to any of claims 1 to 5 for use in the treatment and/or prevention of carious enamel lesions, **characterised in that** the crosslinking monomers having two polymerisable groups are esters of acrylic or methacrylic acid and are preferably selected from the group consisting of allyl methacrylate; allyl acrylate; PRDMA, 1,3-propanediol dimethacrylate; BDMA, 1,3-butanediol dimethacrylate; BDDMA, 1,4-butanediol dimethacrylate; PDDMA, 1,5-pentanediol dimethacrylate; NPGDMA, neopentyl glycol dimethacrylate; HDDMA, 1,6-hexanediol dimethacrylate; NDDMA, 1,9-nonanediol dimethacrylate; DDDMA, 1,10-decanediol dimethacrylate; DDDDMA, 1,12-dodecanediol dimethacrylate; PRDA, 1,3-propanediol diacrylate; BDA, 1,3-butanediol diacrylate; BDDA, 1,4-butanediol diacrylate; PDDA, 1,5-pentanediol diacrylate; NPGDA, neopentyl glycol diacrylate; HDDA, 1,6-hexanediol diacrylate; NDDA, 1,9-nonanediol diacrylate; DDDA, 1,10-decanediol diacrylate; DDDDA, 1,12-dodecanediol dimethacrylate; EGDMA, ethylene glycol dimethacrylate; DEGDMA, diethylene glycol dimethacrylate; TEDMA, triethylene glycol dimethacrylate; TEGDMA, tetraethylene glycol dimethacrylate; EGDA, ethylene glycol diacrylate; DEGDA, diethylene glycol diacrylate; TEDA, triethylene glycol diacrylate; TEGDA, tetraethylene glycol diacrylate; PEG200DMA, polyethylene glycol 200 dimethacrylate; PEG300DMA, polyethylene glycol 300 dimethacrylate; PEG400DMA, polyethylene glycol 400 dimethacrylate; PEG600DMA, polyethylene glycol 600 dimethacrylate; PEG200DA, polyethylene glycol 200 diacrylate; PEG300DA, polyethylene glycol 300 diacrylate; PEG400DA, polyethylene glycol 400 diacrylate; PEG600DA, polyethylene glycol 600 diacrylate; PPGDMA, polypropylene glycol dimethacrylate; PPGDA, polypropylene glycol diacrylate; NPG(PO)2DMA, propoxylated (2) neopentyl glycol dimethacrylate; NPG(PO)2DA, propoxylated (2) neopentyl glycol diacrylate; bis-MA, bisphenol A dimethacrylate; bis-GMA, bisphenol A glycerol dimethacrylate; BPA(EO)DMA, ethoxylated bisphenol A dimethacrylate (EO=1-30); BPA(PO)DMA, propoxylated bisphenol A dimethacrylate (PO=1-30); BPA(EO)DA, ethoxylated bisphenol A diacrylate (EO=1-30); BPA(PO)DA, propoxylated bisphenol A diacrylate (PO=1-30); BPA(PO)GDA, propoxylated bisphenol A glycerol diacrylate; UDMA, diurethane dimethacrylate; TCDDMA, tricyclo[5.2.1.0]decanedimethanol dimethacrylates; TCDDA, tricyclo[5.2.1.0]decanedimethanol diacrylates; EBA, N,N'-ethylenebisacrylamide; DHEBA, N,N'-(1,2-dihydroxyethylene)bisacrylamide; DEPBA, N,N'-diethyl(1,3-propylene)bisacrylamide; TMHMBMA, N,N'-(2,2,4-trimethylhexamethylene)bismethacrylamide; and bis[2-(2-methylacrylamino)ethoxycarbonyl]hexamethylenediamine.

7. Infiltrant according to any of claims 1 to 6 for use in the treatment and/or prevention of carious enamel lesions, **characterised in that** it further comprises monomers having at least three polymerisable groups, the proportion of crosslinking monomers having at least three polymerisable groups being preferably 10 to 50 % by weight, more preferably 10 to 30 % by weight, the crosslinking monomers having at least three polymerisable groups preferably having a distance between crosslinking points of at least 7 bond lengths, more preferably not more than 50 bond lengths, more preferably 10 to 30 bond lengths, more preferably 11 to 21 bond lengths, and more preferably the proportion of crosslinking monomers having at least three polymerisable groups and a distance between crosslinking points of less than 10 bond lengths, based on the total mass of the monomers, being less than 20 % by weight, preferably less than 10 % by weight, more preferably less than 5 % by weight.

8. Infiltrant according to claim 7 for use in the treatment and/or prevention of carious enamel lesions, **characterised in that** the crosslinking monomers having at least three polymerisable groups have the following formula

$$R^1[X_k R^2_1 Y_m]_n$$

having the following definitions:

$R^1$ is a linear or branched hydrocarbon having 3-24 C atoms, containing alkyl, cycloalkyl or aryl; optionally containing O, N, Si, S, P as heteroatoms, for example siloxane and/or cyclosiloxane and/or carbosilane and/or cyclocarbosilane and/or, in particular, ether groups or polyether groups, polyester groups, polysiloxane groups or polycarbosilane groups; optionally substituted by hydroxyl and/or carbonyl and/or halogen (preferably fluorine) and/or ammonium-alkylene groups and/or siloxane and/or cyclosiloxane and/or carbosilane and/or cyclocarbosilane;
$R^2$ is a linear or branched hydrocarbon having 1-16 C atoms, comprising alkyl, cycloalkyl or aryl; optionally containing O, N, Si, S, P as heteroatoms, for example siloxane and/or cyclosiloxane and/or carbosilane and/or cyclocarbosilane and/or, in particular, ether groups or polyether groups, polyester groups, polysiloxane groups or polycarbosilane groups;
optionally substituted by hydroxyl and/or carbonyl and/or halogen (preferably fluorine) and/or ammonium-alkylene groups and/or siloxane and/or cyclosiloxane and/or carbosilane and/or cyclocarbosilane;
X is a linking group identically or differently selected from an ether group, carbonyl group, ester group, amide group, urethane group or urea group;
Y is a group identically or differently containing a polymerisable double bond and/or a ring-openingly polymerisable group and/or a thiol group and/or an epoxide group and/or a vinyl, preferably (meth)acrylate and/or (meth)acrylamide;
$k$ is 0 or 1;
$l$ is 0 or 1;
$m$ is at least 1;
$n$ is at least 1; and
$m \times n$ is at least 3.

9. Infiltrant according to either claim 7 or claim 8 for use in the treatment and/or prevention of carious enamel lesions, **characterised in that** the crosslinking monomers having at least three polymerisable groups are selected from the group consisting of TMPTMA, trimethylolpropane trimethacrylate; TMPTA, trimethylolpropane triacrylate; DTMPTA, ditrimethylolpropane tetra(meth)acrylate; diPENTA, dipentaerythritol penta(meth)acrylate; GPTA, propoxylated glyceryl tri(meth)acrylate; DPEHA, dipentaerythritol hexa(meth)acrylate; and ethoxylated trimethylolpropane tri(meth)acrylate; preferably selected from the group consisting of propoxylated glyceryl tri(meth)acrylate; ethoxylated trimethylolpropane trimethacrylate, ethoxylated trimethylolpropane triacrylate, propoxylated trimethylolpropane trimethacrylate, propoxylated trimethylolpropane triacrylate, ethoxylated pentaerythritol trimethacrylate, ethoxylated pentaerythritol triacrylate, ethoxylated pentaerythritol tetramethacrylate, ethoxylated pentaerythritol tetraacrylate, ethoxylated dipentaerythritol trimethacrylate, ethoxylated dipentaerythritol tetramethacrylate, ethoxylated dipentaerythritol pentamethacrylate, ethoxylated dipentaerythritol hexamethacrylate, ethoxylated dipentaerythritol triacrylate, ethoxylated dipentaerythritol tetraacrylate, ethoxylated dipentaerythritol pentaacrylate, ethoxylated dipentaerythritol hexaacrylate, propoxylated pentaerythritol trimethacrylate, propoxylated pentaerythritol triacrylate, propoxylated pentaerythritol tetramethacrylate, propoxylated pentaerythritol tetraacrylate, propoxylated dipentaerythritol trimethacrylate, propoxylated dipentaerythritol tetramethacrylate, propoxylated dipentaerythritol pentamethacrylate, propoxylated dipentaerythritol hexamethacrylate, propoxylated dipentaerythritol triacrylate, propoxylated dipentaerythritol tetraacrylate, propoxylated dipentaerythritol pentaacrylate and propoxylated dipentaerythritol hexaacrylate.

10. Infiltrant according to any of claims 1 to 9 for use in the treatment and/or prevention of carious enamel lesions, **characterised in that** it has a dynamic viscosity or 50 mPas or less, preferably 30 mPas or less, more preferably 20 mPas or less.

11. Infiltrant according to any of claims 1 to 10 for use in the treatment and/or prevention of carious enamel lesions, **characterised in that** it contains 10 % by weight or less of solvent, more preferably containing essentially no solvent.

12. Kit for preparing an infiltrant according to any of claims 1 to 11 for use in the treatment and/or prevention of carious enamel lesions, **characterised in that** the kit comprises a first component having monomers and chemically activable initiators and a second component having activators, the kit preferably further comprising etching agents and/or drying agents.

**Revendications**

1. Infiltrant pour l'application dentaire, qui contient des monomères réticulants et 20 % en poids ou moins de solvant, **caractérisé en ce que** la proportion de monomères réticulants comportant deux groupes polymérisables est de 99,8 à 50 % en poids, et **en ce qu'**il présente un coefficient de pénétration PK > 100 cm/s et 0,05 à 20 % en poids de monomères contenant des groupes acides, dans lequel les groupes acides sont choisis dans le groupe consistant en groupes d'acide carboxylique, acide sulfonique, acide phosphorique et acide phosphonique, et dans lequel le coefficient de pénétration est défini comme suit :

$$PK = \left( \frac{\gamma . \cos \theta}{2\eta} \right)$$

où

$\gamma$ désigne la tension de surface de la résine fluide (par rapport à l'air)

θ désigne l'angle de contact de la résine fluide (par rapport à l'émail dentaire)

η désigne la viscosité dynamique de la résine fluide ;

pour son utilisation dans le traitement et/ou la prévention de lésions carieuses de l'émail.

2.  Infiltrant selon la revendication 1, pour son utilisation dans le traitement et/ou la prévention de lésions carieuses de l'émail, **caractérisé en ce que** la teneur en monomères contenant des groupes acides est de 0,1 à 15 % en poids, de préférence de 0,2 à 10 % en poids, de manière davantage préférée de 0,5 à 5 % en poids, de manière davantage préférée de 0,5 à 2 % en poids.

3.  Infiltrant selon la revendication 1 ou 2, pour son utilisation dans le traitement et/ou la prévention de lésions carieuses de l'émail, **caractérisé en ce que** les monomères contenant des groupes acides contiennent un ou deux, de préférence un, groupe(s) polymérisable(s).

4.  Infiltrant selon l'une des revendications 1 à 3, pour son utilisation dans le traitement et/ou la prévention de lésions carieuses de l'émail, **caractérisé en ce que** les monomères contenant des groupes acides sont des acrylates, méthacrylates, acrylamides et/ou méthylacrylamide.

5.  Infiltrant selon l'une des revendications 1 à 4, pour son utilisation dans le traitement et/ou la prévention de lésions carieuses de l'émail, **caractérisé en ce que** la proportion de monomères réticulants comportant deux groupes polymérisables est de 99,5 à 60 % en poids, de préférence de 99 à 60 % en poids, de manière davantage préférée, de 99 à 70 % en poids.

6.  Infiltrant selon l'une des revendications 1 à 5, pour son utilisation dans le traitement et/ou la prévention de lésions carieuses de l'émail, **caractérisé en ce que** les monomères réticulants comportant deux groupes polymérisables sont des esters d'acide acrylique ou méthacrylique et sont choisis de préférence dans le groupe comprenant l'allylméthacrylate ; l'allylacrylate ; le PRDMA, le 1,3-propanedioldiméthacrylate ; le BDMA, le 1,3-butanedioldiméthacrylate ; le BDDMA, le 1,4-butanediol-diméthacrylate, le PDDMA, le 1,5-pentanediol-diméthacrylate ; le NPGDMA, le néopentylglycol diméthacrylate ; le HDDMA, le 1,6-hexanediol diméthacrylate ; le NDDMA, le 1,9-nonanediol diméthacrylate ; le DDDMA, le 1,10-décanediol diméthacrylate ; le DDDDMA, le 1,12-dodécanediol diméthacrylate ; le PRDA, le 1,3-propanediol diacrylate ; le BDA, le 1,3-butanediol diacrylate ; le BDDA, le 1,4-butanedioldiacrylate, le PDDA, le 1,5-pentanedioldiacrylate ; le NPGDA, le néopentylglycol diacrylate ; le HDDA, le 1,6-hexanedioldiacrylate ; le NDDA, le 1,9-nonanedioldiacrylate ; le DDDA, le 1,10-décanedioldiacrylate ; le DDDDA, le 1,12-dodécanediol diméthacrylate ; l'EGDMA, l'éthylèneglycol diméthacrylate ; le DEGDMA, le diéthylèneglycol diméthacrylate ; le TEDMA, le triéthylèneglycol diméthacrylate ; le TEGDMA, le tétraéthylèneglycol diméthacrylate ; l'EGDA, l'éthylèneglycoldiacrylate ; le DEGDA, le diéthylèneglycoldiacrylate ; le TEDA, le triéthylèneglycoldiacrylate ; le TEGDA, le tétraéthylèneglycoldiacrylate ; le PEG200DMA, le polyéthylèneglycol 200 diméthacrylate ; le PEG300DMA, le polyéthylèneglycol 300 diméthacrylate ; le PEG400DMA, le polyéthylèneglycol 400 diméthacrylate ; le PEG600DMA, le polyéthylèneglycol 600 diméthacrylate ; le PEG200DA, le polyéthylèneglycol 200 diacrylate ; le PEG300DA, le polyéthylèneglycol 300 diacrylate ; le PEG400DA, le polyéthylèneglycol 400 diacrylate ; le PEG600DA, le polyéthylèneglycol 600 diacrylate ; le PPGDMA, le polypropylène glycoldiméthacrylate ; le PPGDA, le polypropylène glycoldiacrylate ; le NPG(PO)2DMA, le néopentylglycol diméthacrylate (2) propoxylé ; le NPG(PO)2DA, le néopentylglycoldiacrylate (2) propoxylé, le bis-MA, le bisphénol-A-diméthacrylate ; le bis-GMA, le bisphénol-A-glycéroldiméthacrylate ; le BPA(EO)DMA, le bisphénol-A-diméthacrylate éthoxylé (EO=1-30) ; le BPA(PO)DMA, le bisphénol-A-diméthacrylate propoxylé (PO=1-30) ; le BPA(EO)DA, le bisphénol-A-diacrylate éthoxylé (EO=1-30) ; le BPA(PO)DA, le bisphénol-A diacrylate (PO=1-30) propoxylé ; le BPA(PO)GDA, le bisphénol-A-glycérol diacrylate propoxylé ; l'UDMA, le diuréthane diméthacrylate ; le TCDDMA, le tricyclo [5.2.1.0]décane diméthanoldiméthacrylate ; le TCDDA, le tricyclo [5.2.1.0]décanediméthanoldiacrylate ; l'EBA, le N,N'-éthylènebisacrylamide ; le DHEBA, le N,N'-(1,2-dihydroxyéthylène)bisacrylamide ; le DEPBA, le N,N'-diéthyl(1,3-propylène)bisacrylamide ; le TMHMBMA, le N,N'-(2,2,4-triméthylhexaméthylène) bis-méthacrylamide ; et la bis[2-(2-méthylacrylamino) éthoxycarbonyl]hexaméthylènediamine.

7.  Infiltrant selon l'une des revendications 1 à 6, pour son utilisation dans le traitement et/ou la prévention de lésions carieuses de l'émail, **caractérisé en ce qu'**il contient en outre des monomères présentant au moins trois groupes polymérisables, dans lequel la proportion de monomères réticulants comportant au moins trois groupes polymérisables est de préférence de 10 à 50 % en poids, de manière davantage préférée de 10 à 30 % en poids, dans lequel les monomères réticulants comportant au moins trois groupes polymérisables présentent un intervalle des points de réticulation d'au moins 7 longueurs de liaison, de manière davantage préférée d'au plus 50 longueurs de liaison, de manière davantage préférée de 10 à 30 longueurs de liaison, de manière davantage préférée de 11 à 21 longueurs de liaison, et dans lequel, de manière davantage préférée, la proportion de monomères réticulants, comportant au moins trois groupes polymérisables et un intervalle des points de réticulation de moins de 10 longueurs de liaison, est de moins de 20 % en poids, de préférence de moins de 10 % en poids, de manière davantage préférée de moins de 5 % en poids, par rapport à la masse totale des monomères.

8. Infiltrant selon la revendication 7, pour son utilisation dans le traitement et/ou la prévention de lésions carieuses de l'émail, **caractérisé en ce que** les monomères réticulants comportant au moins trois groupes polymérisables présentent la formule suivante :

$$R^1[X_kR^2_lY_m]_n$$

avec les significations suivantes :

$R^1$ est un hydrocarbure linéaire ou ramifié comportant 3 à 24 atomes de C, contenant un alkyle, cycloalkyle ou aryle ;
contenant éventuellement 0, N, Si, S, P comme hétéroatomes, par exemple le siloxane et/ou le cyclosiloxane et/ou le carbosilane et/ou le cyclocarbosilane et/ou en particulier des groupes éther ou polyéther, des groupes polyester, des groupes polysiloxane ou des groupes polycarbosilane ;
éventuellement substitué par un hydroxyle et/ou carbonyle et/ou un halogène (de préférence du fluor) et/ou des groupes ammoniumalkyle et/ou siloxane et/ou cyclosiloxane et/ou carbosilane et/ou cyclocarbosilane ;
$R^2$ est un hydrocarbure linéaire ou ramifié comportant 1 à 16 atomes de C, contenant un alkyle, cycloalkyle ou aryle ;
contenant éventuellement 0, N, Si, S, P comme hétéroatomes, par exemple, le siloxane et/ou le cyclosiloxane et/ou le carbosilane et/ou le cyclocarbosilane et/ou en particulier des groupes éther ou polyéther, des groupes polyester, des groupes polysiloxane ou des groupes polycarbosilane ;
éventuellement substitué par un hydroxyle et/ou carbonyle et/ou un halogène (de préférence du fluor) et/ou des groupes ammoniumalkylène et/ou siloxane et/ou cyclosiloxane et/ou carbosilane et/ou cyclocarbosilane ;
X est un groupe de liaison choisi de façon identique ou différente, dans un groupe éther, un groupe carbonyle, un groupe ester, un groupe amide, un groupe uréthane ou un groupe urée ;
Y est un groupe identique ou différent, contenant une double liaison polymérisable et/ou un groupe polymérisable à cycle ouvert et/ou un groupe thiol et/ou un groupe époxyde et/ou un vinyle ; de préférence, un (méth)acrylate et/ou un (méth)acrylamide ;
k est 0 ou 1 ;
l est 0 ou 1
m est au moins 1
n est au moins 1
m x n est au moins 3.

9. Infiltrant selon la revendication 7 ou 8, pour son utilisation dans le traitement et/ou la prévention de lésions carieuses de l'émail, **caractérisé en ce que** les monomères réticulants comportant au moins trois groupes polymérisables sont choisis dans le groupe comprenant le TMPTMA, le triméthylolpropanetriméthacrylate ; le TMPTA, le triméthylolpropantriacrylate ; le DTMPTA, le ditriméthylolpropanetétra(méth)acrylate ; le DiPENTA, le di-pentaérythritolpenta(méth)acrylate ; le GPTA, le glycéryltri(méth)acrylate propoxylé ; le DPEHA, le dipentaérythritolhexa(méth)acrylate ; et le triméthylol propanetri(méth)acrylate éthoxylé ; sont choisis de préférence dans le groupe comprenant le gycéryltri(méth)acrylate propoxylé ; le triméthylol propanetriméthacrylate éthoxylé, le triméthylolpropanetriacrylate éthoxylé, le triméthylolpropanetriméthacrylate propoxylé, le triméthylolpropanetriacrylate propoxylé, le pentaérythritoltriméthacrylate éthoxylé, le pentaérythritoltriacrylate éthoxylé, le pentaérythritol tétraméthacrylate éthoxylé, le pentaérythritoltétraacrylate éthoxylé, le di-pentaérythritoltriméthacrylate éthoxylé, le di-pentaérythritoltétraméthacrylate éthoxylé, le di-pentaérythritol pentaméthacrylate éthoxylé, le di-pentaérythritol hexaméthacrylate éthoxylé, le di-pentaérythritoltriacrylate éthoxylé, le di-pentaérythritoltétraacrylate éthoxylé, le di-pentaérythritolpentaacrylate éthoxylé, le di-pentaérythritolhexaacrylate éthoxylé, le pentaérythritoltriméthacrylate propoxylé, le pentaérythritoltriacrylate propoxylé, le pentaérythritoltétraméthacrylate propoxylé, le pentaérythritoltétraacrylate propoxylé, le di-pentaérythritoltriméthacrylate propoxylé, le di-pentaérythritoltétraméthacrylate propoxylé, le di-pentaérythritol pentaméthacrylate propoxylé, le di-pentaérythritol hexaméthacrylate propoxylé, le di-pentaérythritoltriacrylate propoxylé, le di-pentaérythritoltétraacrylate propoxylé, le di-pentaérythritolpentaacrylate propoxylé, le di-pentaérythritolhexaacrylate propoxylé.

10. Infiltrant selon l'une des revendications 1 à 9, pour son utilisation dans le traitement et/ou la prévention de lésions carieuses de l'émail, **caractérisé en ce qu'**il présente une viscosité dynamique de 50 mPas ou inférieure, de préférence, de 30 mPas ou inférieure, de manière davantage préférée, de 20 mPas ou inférieure.

11. Infiltrant selon l'une des revendications 1 à 10, pour son utilisation dans le traitement et/ou la prévention de lésions carieuses de l'émail, **caractérisé en ce qu'**il contient 10 % en poids ou moins de solvant, de manière particulièrement préférée, il ne contient essentiellement pas de solvant.

12. Kit de production d'un infiltrant selon l'une des revendications 1 à 11, pour son utilisation dans le traitement et/ou la prévention de lésions carieuses de l'émail, **caractérisé en ce que** le kit contient un premier composant comportant des monomères et des initiateurs activables chimiquement et un deuxième composant comportant des activateurs, dans lequel le kit présente de préférence en outre un agent corrosif et/ou un agent de séchage.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007131725 A1
- EP 1674066 A1
- EP 1974712 A1
- EP 1413569 A
- EP 1741419 A
- EP 1688125 A
- WO 2005086911 A
- WO 02066535 A
- WO 2005121200 A
- WO 02062901 A
- WO 2006031972 A
- EP 1714633 A
- EP 1285947 A
- EP 1849450 A
- US 2004017928 A1
- EP 08014437 A

**In der Beschreibung aufgeführte Nicht-Patentliteratur**

- **BUCKTON G.** Interfacial phenomena in drug delivery and targeting. *Chur,* 1995
- **FAN P. L. et al.** Penetrativity of sealants. *J. Dent. Res.,* 1975, vol. 54, 262-264
- Appl. Sci. Publ. Elsevier, 1984, vol. 2